(19) Europäisches Patentamt
European Patent Office
Office européen des brevets

(11) Veröffentlichungsnummer: **0 122 219**
**B1**

(12) # EUROPÄISCHE PATENTSCHRIFT

(45) Veröffentlichungstag der Patentschrift: **21.09.88**

(51) Int. Cl.⁴: **C 08 F 8/30**

(21) Anmeldenummer: **84810008.7**

(22) Anmeldetag: **06.01.84**

(54) **Resinat einer substituierten Carbonsäure, Verfahren zu seiner Herstellung, seine Verwendung und dieses enthaltende pharmazeutische Präparate.**

(30) Priorität: **12.01.83 CH 147/83**

(43) Veröffentlichungstag der Anmeldung: **17.10.84 Patentblatt 84/42**

(45) Bekanntmachung des Hinweises auf die Patenterteilung: **21.09.88 Patentblatt 88/38**

(84) Benannte Vertragsstaaten: **AT BE CH DE FR IT LI LU NL SE**

(56) Entgegenhaltungen: **DE-A-1 815 802**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Khanna, Satish Chandra, Dr., Rütistrasse 26, CH- 4103 Bottmingen (CH)**

EP 0 122 219 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft das Resinat der Formel

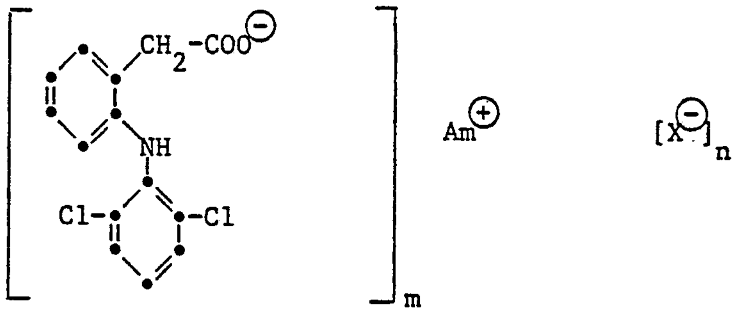

(I)

worin $Am^{\oplus}$ ein in kationischer Form vorliegendes, stark basisches Styrol-Divinylbenzol-Copolymeres bedeutet, das die Summe von m + n quaternären Ammoniumgruppen enthält, und das als Hauptstrukturelement die Gruppierung der Formel

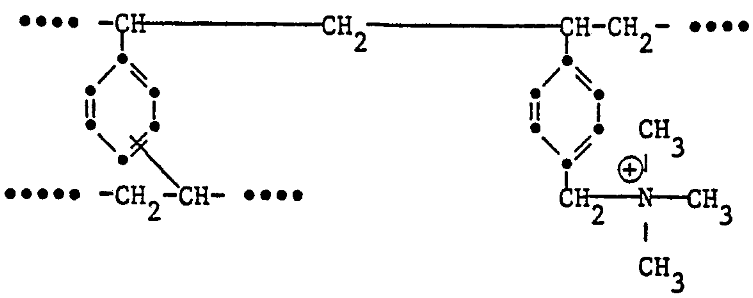

aufweist, $X^{\ominus}$ ein vom Anion der Formel

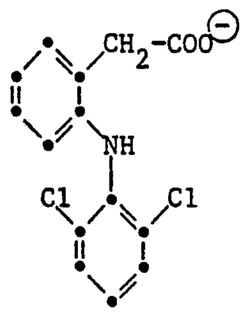

verschiedenes Anion einer Säure bedeutet, und m und n für die ganze Ionenkapazität des Copolymeren steht und dessen Molekulargewicht etwa $10^7$ bis etwa $10^9$ beträgt, Verfahren zu seiner Herstellung, seiner Verwendung als Arzneimittelwirkstoff, sowie pharmazeutische Präparate, die dieses Resinat enthalten.

Die dem Resinat der Formel I zugrundeliegende o-(2,6-Dichloranilino)-phenylessigsäure (Diclofenac) sowie besondere dessen Natriumsalz sind aus der Deutschen Offenlegungsschrift Ns. 1 815 802 bekannt, ebenso deren anti-inflammatorische und analgetische Wirkung. In dem Lehrbuch "Arzneiformenlehre", Dr. P. H. List, 1976, S. 461 und in Remington's Pharmaceutical Science 1975, S. 1626 wird das Prinzip der Resinatbildung beschrieben.

Das Natriumsalz von Diclofenac wird beispielsweise als nicht-steroidales Antiinflammatorikum bei der Behandlung entzündlicher Prozesse eingesetzt. Dabei werden die entsprechenden Präparate überwiegend oral, ferner rektal, topisch oder parenteral, verabreicht.

Diese Präparate vermögen jedoch aus verschiedenen Gründen derzeit nicht voll zu befriedigen: So sind bei oraler Applikationsweise Nebenwirkungen möglich, vor allem im oberen Teil des gastrointestinalen Bereichs. Auch hat das Natriumsalz eine lokalanästhesierende Wirkung auf die Mundschleimhäute und Zunge und ist von bitterem Geschmack. Ebenfalls ist die Wirkungsdauer dieser Präparate begrenzt. Im weiteren ist der im Magen freigesetzte Grundkörper des Wirkstoffes, nämlich die Carbonsäure nur wenig wasserlöslich, was je nach

Mageninhalt zu einer mehr oder weniger langsamen und unregelmässigen Resorption führt.

Diese und weitere bekannte Nachteile des in der Praxis bisher verwendeten Natrium-o-(2,6-dichloranilino)-phenylacetates können durch die Bereitstellung des neuen Resinats der Formel I vermieden oder zumindest erheblich gemildert werden. So ist das erfindungsgemässe Resinat oral wesentlich leichter verabreichbar, da es praktisch geschmacklos ist im Vergleich zum Natriumsalz. Die Geschmacksneutralisierung im Munde ist am besten, wenn das stöchiometrische Verhältnis von Wirkstoff zu Ionenaustauscherharz etwa 1 zu 2 beträgt. Der bevorzugte Verlauf der Wirkstofffreisetzung wird aber am besten mit einem Verhältnis von 1 : 1 erzielt. Obgleich das neue Resinat der Formel I sämtliche erwünschten pharmakologischen Eigenschaften des bekannten Natriumsalzes in zumindest gleicher Wirkungsstärke aufweist, eignet es sich auf Grund seiner spezifischen Vorteile für die orale und rektale Applikation wesentlich besser. So wird der aus dem Resinat freigesetzte Wirkstoff vorwiegend im alkalischen Millieu des Darmtraktes resorbiert, wo er nicht als schwerwasserlösliche Säure, sondern in Form von leicht löslichen Salzen aus dem Resinat freigesetzt wird. Überraschenderweise ergibt sich auch ein für diesen Wirkstoff durchaus erwünschter sogenannter quick-slow release-Effekt im Vergleich zu den bisher bekannten Darreichungsformen, d. h. es erfolgt zuerst eine starke Abgabe des Wirkstoffes und dann eine langsame und nur allmählich abfallende. Überdies ist die Wirkstofffreigabe aus dem erfindungsgemässen Resinat überraschenderweise praktisch unabhängig von der Ionenstärke im Gastrointestinaltrakt, d. h. von seinem Inhalt, der je nach Tageszeit und Ernährungsgewohnheiten verschieden sein kann. Die vorteilhafte Wirkstoffabgabecharakteristik ist aus der Figur ersichtlich.

Die Abgabegeschwindigkeit kann zudem durch die Wahl der Korngrösse des Harzes beeinflusst werden; je grösser die Teilchen, umso geringer ist die Abgabegeschwindigkeit des Wirkstoffes. Die Teilchengrösse liegt vorzugsweise zwischen 20 und 200 µm vorzugsweise 40 bis 100 µm und die Vernetzung zwischen 2 und 8 %, vorzugsweise 2 bis 4 %. Als Cholestyramin-Harz wird vorzugsweise USP-Qualität verwendet.

Das Resinat der Formel I ist dementsprechend vorzüglich als Antiinflammatorikum sowie als Analgetikum zur oralen oder rektalen Applikation verwendbar.

Gegenstand der Erfindung sind ebenso Verfahren zur Herstellung des neuen Resinates der Formel I, welcher nach an sich bekannten Methoden erfolgen kann.

Eine bevorzugte Verfahrensvariante ist beispielsweise dadurch gekennzeichnet, dass man o-(2,6-Dichloranilino)-phenylessigsäure der Formel

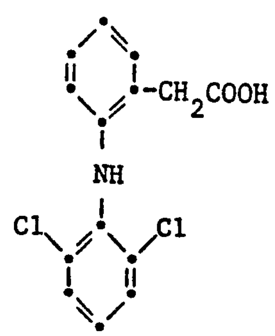

(II)

oder ein Salz davon mit vorzugsweise der mindestens äquimolaren Menge Harz der Formel $[Am^{\oplus}]\,[OH^{\ominus}]_{m+n}$ oder einem Salz desselben mit einer Säure, vorzugsweise einer Mineralsäure, umsetzt.

Verfahrensgemäss verwendbare Anionenaustauscherharze sind insbesondere Cholestyramin, wie es z. B. unter der Markenbezeichnung Duolite® A 101D, A 101 D/U, A 102D, A 113, A 116, 143 A 161, A 162 und ES 132 von Diamond Shamrock oder Amberlite® XE 268 P von Rohm und Haas erhältlich sind und einen Polymerisationsgrad von ungefähr $10^8$ aufweisen.

Verfahrensgemäss verwendbare Salze der Säure der Formel II sind insbesondere Salze mit Basen, die aus dem Reaktionsgemisch entfernt werden können, beispielsweise anorganische Salze, wie z. B. Alkalisalze.

Die Erfindung betrifft weiterhin pharmazeutische Präparate, enthaltend das Resinat der Formel I bzw. ein Verfahren zur Herstellung pharmazeutischer Präparate. Das Verfahren ist dadurch gekennzeichnet, dass man das Resinat der Formel I mit üblichen Hilfs- und/oder Zusatzstoffen vermischt und zu einer galenischen Form verarbeitet.

Die Erfindung betrifft dabei namentlich die in den Beispielen beschriebenen pharmazeutischen Präparate und Verfahren zu deren Herstellung.

Bei den erfindungsgemässen pharmazeutischen Präparaten, welche das Resinat der Formel I enthalten, handelt es sich um solche zur enteralen, wie oralen oder rektalen, Verabreichung.

Die neuen pharmazeutischen Präparate sind vorzugsweise pharmazeutische Präparate zur enteralen Verabreichung, z. B. solche in Doseneinheitsformen wie orale und rektale Präparate, z. B. Dragées, Tabletten, Kapseln, Sirupe, Tropfen, Suppositorien oder Rektalkapseln.

Die Präparate werden in an sich bekannter Weise, z. B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoffkomplex mit festen Trägerstoffen kombiniert, ein erhaltenes

Gemisch gegebenenfalls granuliert, und das Gemisch bzw. Granulat, wenn erwünscht oder notwendig, nach Zugabe von geeigneten Hilfsstoffen, zu Tabletten oder Dragées verarbeitet.

Geeignete Trägerstoffe sind insbesondere Füllstoffe, wie Zucker, z. B. Lactose, Saccharose, Mannit oder Sorbit, Cellulosepräparate und/oder Calciumphosphate, z. B. Tricalciumphosphat oder Calciumhydrogenphosphat, ferner Bindemittel, wie Stärkekleister unter Verwendung z. B. von Mais-, Weizen-, Reis oder Kartoffelstärke, Gelatine, Traganth, Methylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie *Natriumalginat*. Hilfsmittel sind in erster Linie Fliessregulier- und Schmiermittel, z. B. Kieselsäure, Talk, Stearinsäure oder Salze davon, wie Magnesium- oder Calciumstearat und/oder Polyäthylenglykol. Dragée-Kerne werden mit geeigneten Überzügen versehen, wobei man u. a. konzentrierte Zuckerlösungen, welche gegebenenfalls arabischen Gummi, Talk, Polyvinylpyrrolidon, Polyäthylenglycol und/oder Titandioxid enthalten. Den Tabletten oder Dragée-Überzügen können Farbstoffe oder Pigmente, z. B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoffkomplex in Form eines Granulates, z. B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff-Komplex vorzugsweise in geeigneten Flüssigkeiten, wie Fetten Ölen, Paraffinöl oder flüssigen Polyäthylenglycolen, gelöst oder suspendiert, wobei ebenfalls Stabilisatoren zugefügt sein können.

Orale Verbindungsformen sind auch Trinksuspensionen in Form von Sirupen.

Da das erfindungsgemässe Resinat sich bei der Verarbeitung und der Lagerung, selbst in Form eines pharmazeutischen Präparates, in geringem Masse zersetzt, wobei noch in grosser Verdünnung unangenehm riechende aliphatische Amine auftreten, werden ihm vorteilhafterweise geruchsbindende Substanzen beigemischt. Dabei bewähren sich besonders Aktivkohle oder Kationenaustauscher auf Styrol-Divinylbasis mit Sulfonsäure- oder Carboxylgruppen (siehe DDR Patentschrift 147 819).

Als rektal anwendbare pharmazeutische Präparate kommen z. B. Suppositorien in Betracht, welche aus einer Kombination des Wirkstoffs mit einer Suppositoriengrundmasse bestehen. Als Suppositoriengrundmasse eignen sich z. B. natürliche oder synthetische Triglyceride, Paraffinkohlenwasserstoffe, Polyäthylenglycole oder höhere Alkanole. Ferner können auch Gelatine-Rektalkapseln verwendet werden, die eine Kombination des Wirkstoffkomplexes mit einer Grundmasse enthalten; als Grundmassenstoffe kommen z. B. flüssige Triglyceride, Polyäthylenglykole oder Paraffinkohlenwasserstoff in Frage.

Die vorliegende Erfindung betrifft ebenfalls die Verwendung des Resinats der Formel I, vorzugsweise zur Behandlung von Entzündungen, in erster Linie von entzündlichen chronischen Erkrankungen des rheumatischen Formenkreises, besonders der chronischen Arthritis.

Die nachfolgenden Beispiele illustrieren die oben beschriebene Erfindung. Temperaturen sind in Celsiusgraden, Drucke in mbar angegeben.

**Beispiel 1:**

Konditionierung des Harzes: 100 g Cholestyramin (Duolite® 143) mit einer Korngrösse von 40 oder 80 µm werden in 500 ml 2N Natriumhydroxid suspendiert. Die Mischung wird während 4 Stunden bei 50° gerührt. Die überstehende Lösung wird abdekantiert und das Harz 4 mal mit deionisiertem Wasser gewaschen. Dann werden 500 ml 2N Salzsäure zugegeben und die Mischung wieder während 4 Stunden bei 50° gerührt. Die überstehende Flüssigkeit wird wieder abdekantiert und das Harz mit einem Überschuss von heissem deionisiertem Wasser gewaschen bis der pH der abdekantierten Lösung zwischen 8 und 9 liegt. Danach wird das Harz während 2 Stunden in Isopropylalkohol suspendiert um mögliche organische Verunreinigungen zu entfernen. Danach wird das Harz filtriert und 2 mal mit deionisiertem Wasser gewaschen. Die Trocknung zur Gewichtskonstanz erfolgt bei 50° im Vakuum.

a) Beladung des Harzes mit Wirkstoff: 100 g Diclofenac-natrium werden in 5 Liter deionisiertem Wasser aufgelöst und dann 100 g konditioniertes Cholestyramin (80 µm) langsam in dieser Lösung verteilt. Die Mischung wird während ca. 12 Stunden bei 50° gerührt. Das erhaltene Wirkstoffresinat wird abfiltriert und bis zur Gewichtskonstanz bei 50° im Vakuum getrocknet.

b) Beladung des Harzes mit Wirkstoff: 100 g Diclofenac-natrium werden in 5 Liter deionisiertem Wasser aufgelöst und dann 200 g konditioniertes Cholestyremin (40 µm) langsam in dieser Lösung verteilt. Die Mischung wird während ca. 12 Stunden bei 50° gerührt. Das erhaltene Wirkstoffresinat wird abfiltriert und bis zur Gewichtskonstanz bei 50° im Vakuum getrocknet.

**Beispiel 2:**

Tabletten enthaltend Wirkstoff entsprechend 150 mg Diclofenac-Na können folgendermassen hergestellt werden:

| Bestandteile (für 1000 Tabletten) | |
|---|---|
| Wirkstoff-Resinat gemäss Beispiel 1a | 300g |
| Lactose | 100,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb von 0,6 mm Maschenweite getrieben. Dann werden das Wirkstoffresinat, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben und mit der entstandenen Suspension das obige Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig gewölbte Tabletten von etwa 8 mm Durchmesser verpresst.

**Beispiel 3:**

Tabletten enthaltend Wirkstoff entsprechend 150 mg Diclofenac-Na können folgendermassen hergestellt werden:

| Bestandteile (für 20'000 Tabletten) | |
|---|---|
| Wirkstoff-Resinat gemäss Beispiel 1a | 3000g |
| Lactose, gemahlen | 350,0 g |
| kolloidales Siliciumdioxid | 30,0 g |
| Polyvinylpyrrolidon | 30,0 g |
| mikrokristalline Cellulose | 400,0 g |
| Maisstärke | 690,0 g |
| Zerolit® 236 SRC 48 | 100,0 g |

Herstellung: Sämtliche festen Ingredienzien werden zunächst durch ein Sieb von 0,6 mm Maschenweite getrieben. Dann wird das Wirkstoff-Resinat mit allen Hilfsstoffen vermischt. Die fertige Tabletten-Grundmasse wird alsdann zu Tabletten von 9 mm Durchmesser und 230 mg Gewicht verpresst.

**Beispiel 4:**

2000 g Wirkstoff-Resinat gemäss Beispiel 1a werden in 10 000 Kapseln (Grösse 1) abgefüllt. Jede Kapsel enthält 100 mg Wirkstoff.

**Beispiel 5:**

3 000 g Wirkstoff-Resinat gemäss Beispiel 1a werden mit 10 g Aktivkohle gründlich vermischt und durch ein Sieb von 0,6 mm Maschenweite getrieben. Danach wird die Mischung in 10 000 Kapseln (Grösse 1) abgefüllt. Jede Kapsel enthält 150 mg Wirkstoff.

**Beispiel 6:**

20 g Traganth, 6 g p-Hydroxbenzoesäuremethylester und 1,5 g p-Hydroxybenzeosäurepropylester werden in 2 Liter Wasser bei 80 - 90°C aufgelöst. Das Erhaltene Gel wird abgekühlt und 75 g des oben erhaltenen, trockenen Wirkstoff-Resinat gemäss Beispiel 1b) und 5 g Zerolit 225 (Teilchengrösse 50 µ) werden zugefügt und unter Verwendung eines Homogenisators gründlich dispergiert. 2 000 g Sorbit-Lösung 70 % werden

zugesetzt. Dann wird der Dispersion Wasser bis zum Endvolumen von 5 Liter zugefügt, so dass die erhaltene Suspension etwa 1,5 % Resinat enthält. Ein Teelöffel dieser Suspension enthält ungefähr eine Dosis, die 50 mg Diclofenacnatrium entspricht. Genau ist diese Dosis in 5 ml Suspension enthalten.

**Beispiel 7:**

3 g des gemäss Beispiel 1a erhaltenen Diclofenac-Resinat werden in einem geschmolzenen Gemisch von 20 g Polyäthylenglykol 4000 und Polyäthylenglykol 1000 (1 : 1) suspendiert. Die erhaltene Schmelzmasse wird in Suppositorienformen gegossen und dann abgekühlt. Jedes Rektal-Suppositorium wiegt ca. 2 g und enthält Wirkstoff entsprechend 150 mg Diclofenac-Na.

**Beispiel 8:**

60 g Duolite® A 161 (durchschnittliche Teilchengrösse: 80 μm) werden in einem Liter 1,5 N Natriumhydroxid suspendiert, und die Suspension wird auf einem Wasserbad auf 50° erhitzt. Nach 4 bis 5 Stunden wird die Suspension filtriert und das Filtrat mit deionisiertem Wässer gewaschen. Das so erhaltene Harz wird in einem Liter 2N Salzsäure suspendiert und bei 50° während 4 bis 5 Stunden gerührt. Das Harz wird abfiltriert und das Filtrat mit deionisiertem Wasser gewaschen. Die oben erwähnte Schritte werden zweimal wiederholt.

Das erhaltene Harz wird in Isopropylalkohol suspendiert und während 4 bis 5 Stunden gerührt. Das abfiltrierte Harz wird bei 50° im Vakuum getrocknet.

15 g Diclofenac-Natrium werden in einem Liter deionisiertem Wasser gelöst. 15 g des erhaltenen aktivierten Harzes werden in der hergestellten Lösung suspendiert und die Suspension während 12 Stunden gerührt. Danach wird das erhaltene Resinat abfiltriert und bei 50° unter Vakuum getrocknet.

Aus diesem Resinat können Tabletten, Kapseln, Suspensionen und Suppositorien gemäss den Angaben in den Beispielen 2 bis 6 hergestellt werden.

**Patentansprüche** für die Vertragsstaaten BE, CH, DE, FR, IT, LI, LU, NL, SE

1. Resinat der Formel

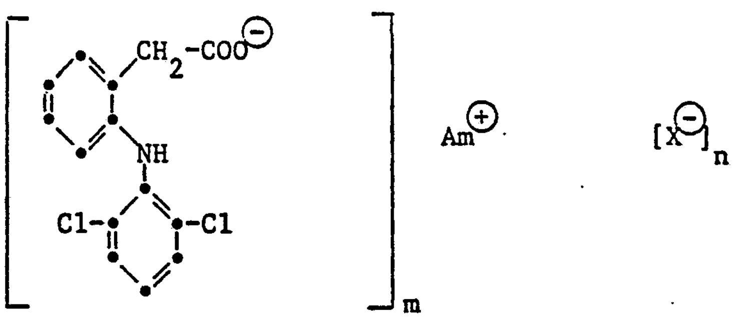 (I)

worin Am⊕ ein in kationischer Form vorliegendes, stark basisches Styrol-Divinylbenzol-Copolymeres bedeutet, das die Summe von m + n quaternären Ammoniumgruppen enthält, und das als Hauptstrukturelement die Gruppierung der Formel

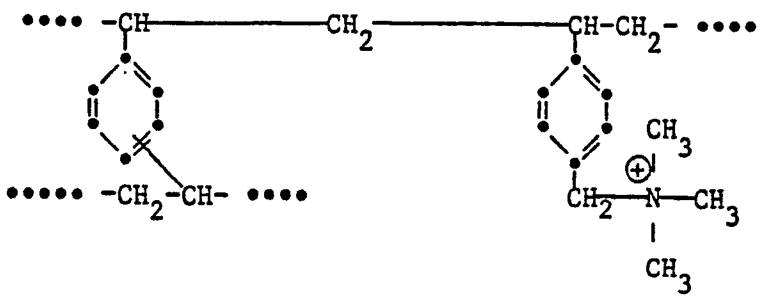

aufweist, $X^{\ominus}$ ein vom Anion der Formel

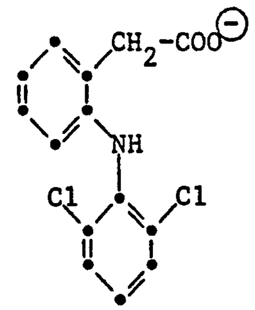

verschiedenes Anion einer Säure bedeutet, und m und n für die ganze Ionenkapazität des Copoylmeren steht und dessen Molekulargewicht etwa $10^7$ bis etwa $10^9$ beträgt.

2. Resinat gemäss Anspruch 1, dadurch gekennzeichnet, dass das stöchiometrische Verhältnis von Wirkstoff zu Ionenaustauscherharz etwa 1 : 1 bis 1 : 2 beträgt.

3. Resinat gemäss Anspruch 1 und 2, dadurch gekennzeichnet, dass die Teilchengrösse des Harzes zwischen 20 und 200 µm liegt.

4. Resinat gemäss Anspruch 3, dadurch gekennzeichnet, dass die Teilchengrösse des Harzes zwischen 40 und 100 µm liegt.

5. Resinat gemäss Anspruch 1 - 4, dadurch gekennzeichnet, dass die Vernetzung des Harzes zwischen 2 und 8 % liegt.

6. Resinat gemäss Anspruch 5, dadurch gekennzeichnet, dass die Vernetzung des Harzes zwischen 2 bis 4 % liegt.

7. Resinat gemäss Anspruch 1 - 6, dadurch gekennzeichnet, dass als Anionenaustauscherpolymeres Cholestyramin verwendet wird.

8. Resinat gemäss Anspruch 1 - 7, dadurch gekennzeichnet, dass als Styrol-Divinylbenzol-Copolymeres Cholestyramin mit einem Polymerisationsgrad von ungefähr $10^8$ verwendet wird.

9. Resinat der Formel I gemäss Anspruch 1 zur Anwendung bei der Behandlung des menschlichen oder tierischen Körpers.

10. Pharmazeutisches Präparat enthaltend das Resinat der Formel I gemäss Anspruch 1.

11. Verwendung des Resinates der Formel I gemäss Anspruch 1 zur Herstellung von pharmazeutischen Präparaten.

12. Verfahren zur Herstellung eines Resinates gemäss Anspruch 1 - 8, dadurch gekennzeichnet, dass man o-(2,6-Dichloranilino)-phenylessigsäure oder ein Salz davon mit einem Harz der Formel

$[Am^{\oplus}]\,[OH^{\ominus}]_{m+n}$

oder einem Salz desselben mit einer Säure umsetzt.

**Patentansprüche** für den Vertragsstaat AT

1. Verfahren zur Herstellung des Resinats der Formel

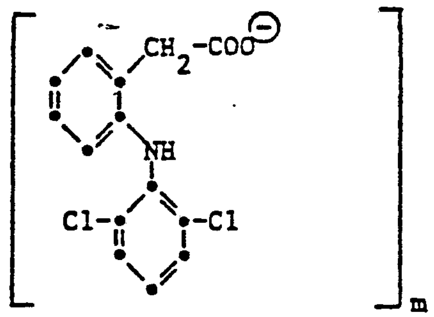

$Am^{\oplus}$ $[X^{\ominus}]_n$ (I)

worin $Am^{\oplus}$ ein in kationischer Form vorliegendes, stark basisches Styrol-Divinylbenzol-Copolymeres bedeutet, das die Summe von m + n quaternären Ammoniumgruppen enthält, und das als Hauptstrukturelement die Gruppierung der Formel

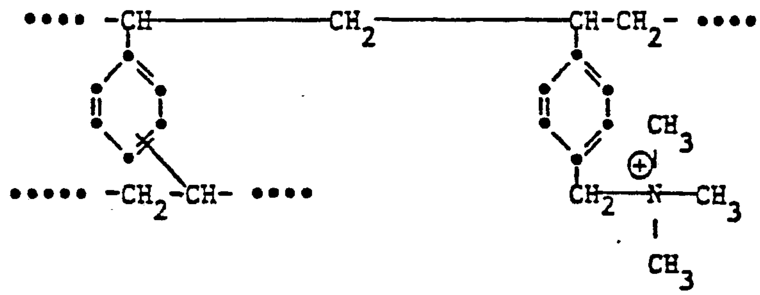

aufweist, $X^{\ominus}$ ein vom Anion der Formel

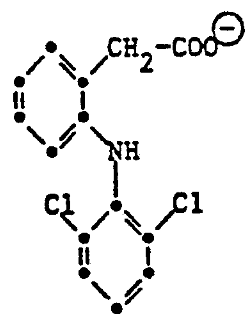

verschiedenes Anion einer Säure bedeutet, und m und n für die ganze Ionenkapazität des Copoylmeren steht und dessen Molekulargewicht etwa $10^7$ bis etwa $10^9$ beträgt, dadurch gekennzeichnet, dass man o-(2,6-Dichloranilino)-phenylessigsäure oder ein Salz davon mit einem Harz der Formel

$[Am^{\oplus}]\ [OH^{\ominus}]_{m+n}$

oder einem Salz desselben mit einer Säure umsetzt.

2. Verfahren gemäss Anspruch 1, dadurch gekennzeichnet, dass das stöchiometrische Verhältnis von Wirkstoff zu Ionenaustauscherharz etwa 1 : 1 bis 1 : 2 beträgt.

3. Verfahren nach Anspruch 1 oder 2, dadurch gekennzeichnet, dass die Teilchengrösse des Harzes zwischen 20 und 200 µm liegt.

4. Verfahren nach Anspruch 3, dadurch gekennzeichnet, dass die Teilchengrösse des Harzes zwischen 40 und 100 µm liegt.

5. Verfahren nach einem der Ansprüche 1 - 4, dadurch gekennzeichnet, dass die Vernetzung des Harzes zwischen 2 und 8 % liegt.

6. Verfahren nach Anspruch 5, dadurch gekennzeichnet, dass die Vernetzung des Harzes zwischen 2 und 4 % liegt.

7. Verfahren nach einem der Ansprüche 1 - 6, dadurch gekennzeichnet, dass als Anionenaustauscherpolymeres Cholestyramin verwendet wird.

8. Verfahren nach einem der Ansprüche 1 - 7, dadurch gekennzeichnet, dass als Styrol-Divinylbenzol-Copolymeres Cholestyramin mit einem Polymerisationsgrad von ungefähr $10^8$ verwendet wird.

9. Verfahren zur Herstellung von pharmazeutischen Präparaten, dadurch gekennzeichnet, dass man ein Resinat gemäss einem der Ansprüche 1 - 8, gegebenenfalls unter Beimischung von üblichen Hilfsstoffen, zu pharmazeutischen Präparaten verarbeitet.

**Claims** for the Contracting States: BE CH DE FR IT LI LU NL SE

1. A resinate of the formula

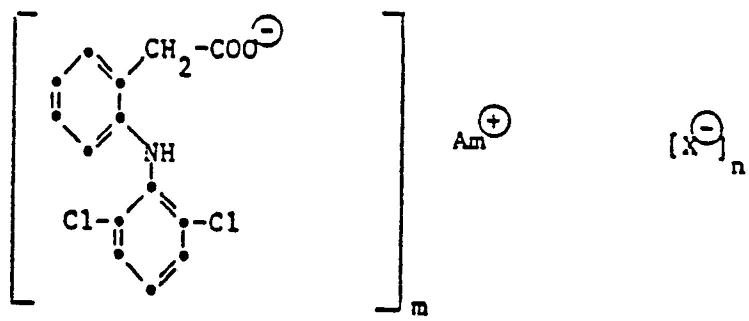

(I)

wherein $Am^{\oplus}$ is a strongly basic copolymer of styrene and divinylbenzene which is in cationic form, said copolymer containing the sum of m + n quaternary ammonium groups and also containing, as main structural unit, the grouping of the formula

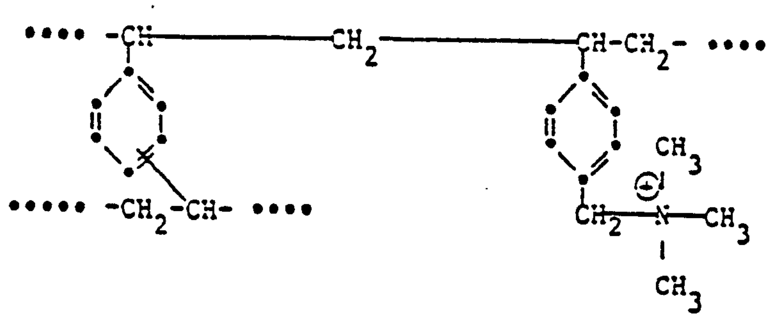

$X^{\ominus}$ is an anion of an acid different from the anion of the formula

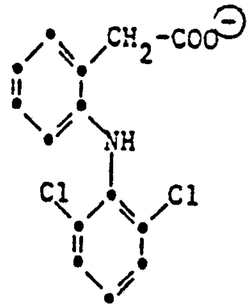

and m and n denote the entire ionic capacity of the copolymer, and the molecular weight of which is about $10^7$ to about $10^9$.

2. A resinate according to claim 1, wherein the stoichiometric ratio of active ingredient to ion exchange resin is about 1 : 1 to 1 : 2.

3. A resinate according to claims 1 and 2, wherein the particle size of the resin is between 20 and 200 μm.

4. A resinate according to claim 3, wherein the particle size of the resin is between 40 and 100 μm.

5. A resinate according to claims 1 - 4, wherein the crosslinkage of the resin is between 2 and 8 %.

6. A resinate according to claim 5, wherein the crosslinkage of the resin is between 2 and 4 %.

7. A resinate according to claims 1 - 6, wherein cholestyramine is used as the anion exchange polymer.

8. A resinate according to claims 1 - 7, wherein cholestyramine having a degree of polymerisation of

approximately $10^8$ is used as the copolymer of styrene and divinylbenzene.

9. A resinate of the formula I according to claim 1 for use in the treatment of the human or animal body.

10. A pharmaceutical composition which contains a resinate of the formula I according to claim 1.

11. Use of a resinate of the formula I according to claim 1 for the preparation of pharmaceutical compositions.

12. A process for the preparation of a resinate according to claims 1-8, which comprises reacting o-(2,6-dichloroanilino)phenylacetic acid, or a salt thereof, with a resin of the formula

$$[Am^{\oplus}]\,[OH^{\ominus}]_{m+n}$$

or a salt thereof with an acid.

**Claims** for the Contracting State AT

1. A process for the preparation of a resinate of the formula

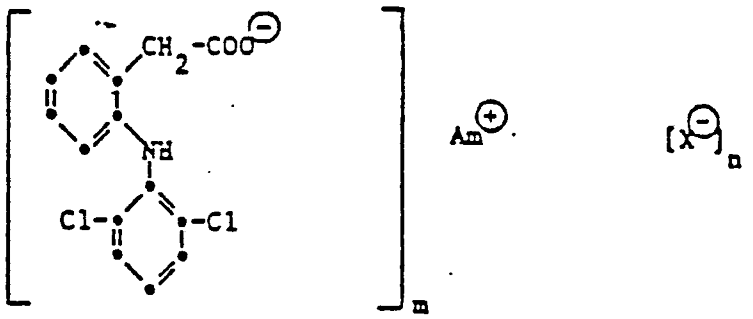

(I)

wherein $Am^{\oplus}$ is a strongly basic copolymer of styrene and divinylbenzene which is in cationic form, said copolymer containing the sum of m + n quaternary ammonium groups and also containing, as main structural unit, the grouping of the formula

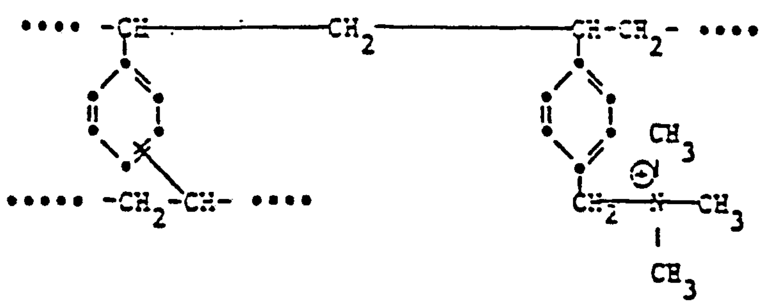

$X^{\ominus}$ is an anion of an acid different from the anion of the formula

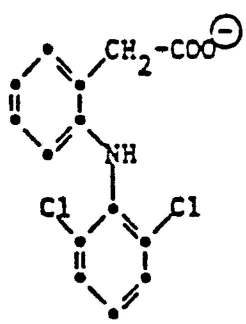

and m and n denote the entire ionic capacity of the copolymer, and the molecular weight of which is about $10^7$ to about $10^9$, which comprises reacting o-(2,6-dichloroanilino)phenylacetic acid, or a salt thereof, with a

resin of the formula

$[Am^{\oplus}]\,[OH^{\ominus}]_{m+n}$

or a salt thereof with an acid.

2. A process according to claim 1, wherein the stoichiometric ratio of active ingredient to ion exchange resin is about 1 : 1 to 1 : 2.

3. A process according to claim 1 or 2, wherein the particle size of the resin is between 20 and 200 μm.

4. A process according to claim 3, wherein the particle size of the resin is between 40 and 100 μm.

5. A process according to any one of claims 1 - 4, wherein the crosslinkage of the resin is between 2 and 8 %.

6. A process according to claim 5, wherein the crosslinkage of the resin is between 2 and 4 %.

7. A process according to any one of claims 1 - 6, wherein cholestyramine is used as the anion exchange polymer.

8. A process according to any one of claims 1 - 7, wherein cholestyramine having a degree of polymerisation of approximately $10^8$ is used as the copolymer of styrene and divinylbenzene.

9. A process for the preparation of pharmaceutical compositions, wherein a resinate according to any one of claims 1 - 8 is processed to form pharmaceutical compositions, optionally with the admixture of conventional adjuncts.

**Revendications** pour les Etats contractants BE, CH, DE, FR, IT, LI, LU, NL, SE

1. Résinate de formule

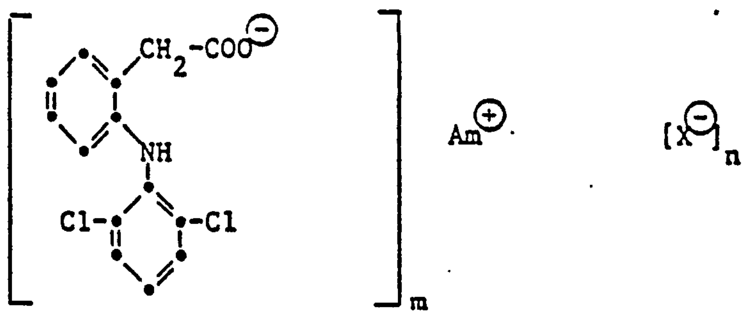

(I)

dans laquelle $Am^{\oplus}$ représente un copolymère styrène-divinylbenzène fortement basique sous forme cationique, contenant la somme de m + n groupes ammonium quaternaire, et qui presente comme élément de structure principal le groupement de formule

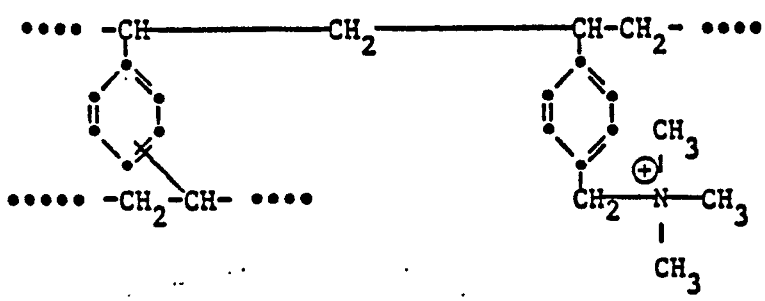

$X^{\ominus}$ représente un anion d'acide différent de l'anion de formule

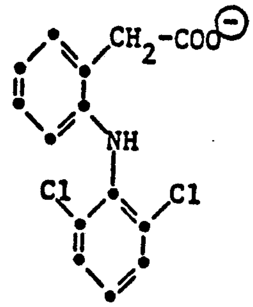

et m et n représentent la capacité ionique totale du copolymère, et dont le poids moléculaire est d'environ $10^7$ à $10^9$.

2. Résinate selon la revendication 1, caractérisé en ce que le rapport stoechiométrique entre la substance active et la résine échangeuse d'ions est d'environ 1 : 1 à 1 : 2.

3. Résinate selon les revendications 1 et 2, caractérisé en ce que la dimension de particule de la résine se situe entre 20 et 200 µm.

4. Résinate selon la revendication 3, caractérisé en ce que la dimension de particule de la résine se situe entre 40 et 100 µm.

5. Résinate selon les revendications 1 à 4, caractérisé en ce que le taux de réticulation de la resine est de 2 à 8 %.

6. Résinate selon la revendication 5, caractérisé en ce que le taux de réticulation de la résine est de 2 à 4 %.

7. Résinate selon les revendications 1 à 6, caractérisé en ce que l'on utilise en tant que polymère échangeur d'anions la cholestyramine.

8. Résinate selon les revendications 1 à 7, caractérisé en ce que l'on utilise en tant que copolymère styrène-divinylbenzène la cholestyramine à un degré de polymérisation d'environ $10^8$.

9. Résinate de formule I selon la revendication 1, pour l'utilisation dans le traitement de l'organisme humain ou animal.

10. Composition pharmaceutique contenant le résinate de formule I selon la revendication 1.

11. Utilisation du résinate de formule I selon la revendication 1 pour la préparation de compositions pharmaceutiques.

12. Procédé de préparation d'un résinate selon les revendications 1 à 8, caractérisé en ce que l'on fait réagir l'acide o-(2,6-dichloranilino)-phénylacétique ou l'un de ses sels avec une résine de formule

$$[Am^{\oplus}]\ [OH^{\ominus}]_{m+n}$$

ou un sel de cette résine et d'un acide.

**Revendications** pour L'Etat contractant AT

1. Procédé de préparation du résinate de formule

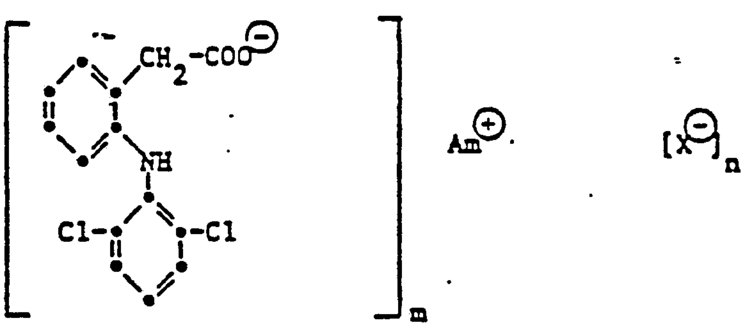

(I)

dans laquelle Am⊕ représente un copolymère styrène-divinylbenzène fortement basique sous forme cationique, contenant la somme de m + n groupes ammonium quaternaire et dont l'élément de structure principal est le groupement de formule

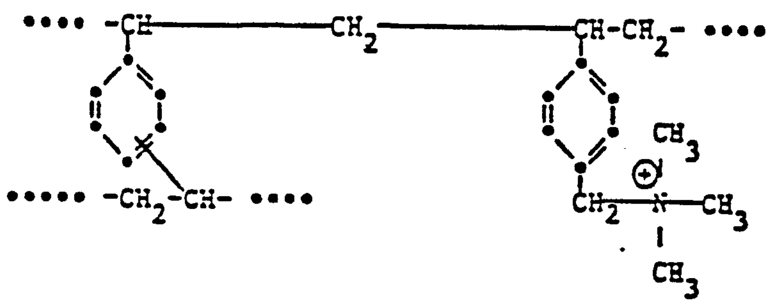

$X^{\ominus}$ représente un anion d'acide différent de l'anion de formule

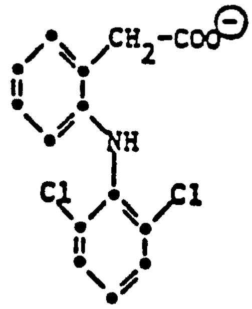

et m et n représentent la capacité ionique totale du copolymère, et dont le poids moléculaire est d'environ $10^7$ à $10^9$, caractérisé en ce que l'on fait réagir l'acide o-(2,6-dichloranilino)-phénylacétique ou l'un de ses sels avec une résine de formule

$[Am^{\oplus}]\ [OH^{\ominus}]_{m+n}$

ou un sel de cette résine et d'un acide.

2. Procédé selon la revendication 1, caracterisé en ce que le rapport stoechiométrique entre la substance active et la résine échangeuse d'ions est d'environ 1 : 1 à 1 : 2.

3. Procédé selon la revendication 1 ou 2, caractérisé en ce que la dimension de particule de la resine se situe entre 20 et 200 µm.

4. Procédé selon la revendication 3, caractérisé en ce que la dimension de particule de la résine se situe entre 40 et 100 µm.

5. Procédé selon l'une des revendications 1 à 4, caractérisé en ce que le taux de réticulation de la résine se situe entre 2 et 8 %.

6. Procédé selon la revendication 5, caractérisé en ce que le taux de réticulation de la résine se situe entre 2 et 4 %.

7. Procédé selon l'une des revendications 1 à 6, caractérisé en ce que l'on utilise en tant que polymère échangeur d'anions la cholestyramine.

8. Procédé selon l'une des revendications 1 à 7, caractérisé en ce que l'on utilise en tant que copolymère styrène-divinylbenzène la cholestyramine à un degré de polymérisation d'environ $10^8$.

9. Procédé de préparation de compositions pharmaceutiques, caractérisé en ce que l'on met un résinate selon l'une des revendications 1 à 8, le cas échéant après mélange avec des produits auxiliaires usuels, sous la forme de compositions pharmaceutiques.

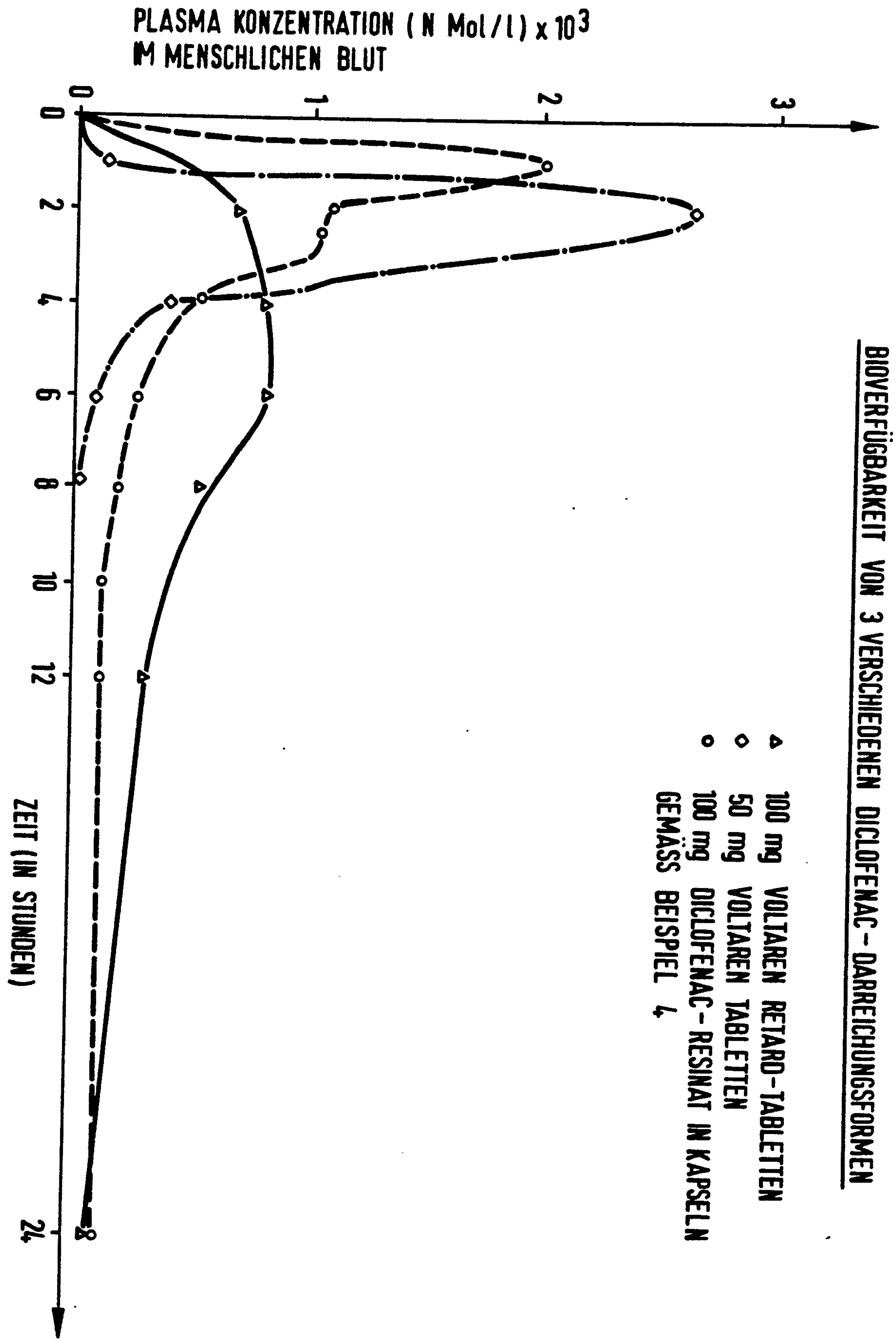
PLASMA KONZENTRATION ( N Mol / l ) x 10³
IM MENSCHLICHEN BLUT
0
1
2
3
0
2
4
6
8
10
12
24
ZEIT (IN STUNDEN)
BIOVERFÜGBARKEIT VON 3 VERSCHIEDENEN DICLOFENAC- DARREICHUNGSFORMEN
100 mg VOLTAREN RETARD-TABLETTEN
50 mg VOLTAREN TABLETTEN
100 mg DICLOFENAC-RESINAT IN KAPSELN GEMÄSS BEISPIEL 4